(19) **Europäisches Patentamt / European Patent Office / Office européen des brevets**

(11) **EP 1 333 850 B1**

(12) **EUROPEAN PATENT SPECIFICATION**

(45) Date of publication and mention
of the grant of the patent:
**20.08.2008 Bulletin 2008/34**

(51) Int Cl.:
*A61K 38/00* (2006.01)       *A61P 35/00* (2006.01)

(21) Application number: **01989341.1**

(86) International application number:
**PCT/US2001/051339**

(22) Date of filing: **22.10.2001**

(87) International publication number:
**WO 2002/055100 (18.07.2002 Gazette 2002/29)**

(54) **USE OF IL-13 INHIBITORS FOR THE TREATMENT OF TUMORS**

VERWENDUNG VON IL-13 INHIBITOREN ZUR BEHANDLUNG VON TUMOREN

UTILISATION DES INHIBITEURS DE L'IL-13 POUR LE TRAITEMENT DE TUMEURS

(84) Designated Contracting States:
**AT BE CH CY DE DK ES FI FR GB GR IE IT LI LU MC NL PT SE TR**

(30) Priority: **20.10.2000 US 693600**
**07.09.2001 US 318185 P**

(43) Date of publication of application:
**13.08.2003 Bulletin 2003/33**

(73) Proprietors:
• **GENETICS INSTITUTE, INC.**
**Cambridge,**
**Massachusetts 02140 (US)**
• **THE GOVERNMENT OF THE UNITED STATES OF AMERICA, as**
**represented by THE SECRETARY, DEPARTMENT OF HEALTH**
**AND HUMAN SERVICES**
**Rockville, MD 20852 (US)**

(72) Inventors:
• **BERZOFSKY, Jay, A.**
**Bethesda, MD 20814 (US)**
• **TERABE, Masaki**
**Bethesda, MD 20814 (US)**
• **DONALDSON, Debra, D.**
**Medford, MA 02155 (US)**
• **MATSUI, So**
**Tsukuba, Ibariki 300-0051 (JP)**
• **NOBEN-TRAUTH, Nancy**
**Rockville, MD 20855 (US)**
• **PAUL, William, E.**
**Potomac, MD 20851 (US)**

(74) Representative: **Dörries, Hans Ulrich**
**df-mp**
**Fünf Höfe**
**Theatinerstrasse 16**
**80333 München (DE)**

(56) References cited:
EP-A- 1 176 140          WO-A-00/36103
WO-A-00/40264          WO-A-01/77332
WO-A-94/04680          US-A- 6 143 871

• **TRIEU Y, WEN X-Y, SKINNIDER B F, BRAY M R, LI Z, CLAUDIO J O, MASIH-KHAN E, ZHU Y-X, TRUDEL S, MCCART J A, MAK T W, STEWART A K: "Soluble interleukin-13Ralpha2 decoy receptor inhibits Hodgkin's lymphoma growth in vitro and in vivo" CANCER RESEARCH, vol. 64, 1 May 2004 (2004-05-01), pages 3271-3275,**
• **KAWAKAMI K: "Cancer gene therapy utilizing interleukin-13 receptor alpha2 chain" CURRENT GENE THERAPY, vol. 5, 2005, pages 213-223,**

Remarks:
The file contains technical information submitted after the application was filed and not included in this specification

**Description**

**Field of the Invention**

The invention relates generally to compositions for inhibiting the growth of a tumor by inhibiting IL-13 expression or activity and/or NK-T cell activity.

**Background of the Invention**

The cytokine IL-13 has been implicated in several biological activities. These activities include, *e.g.*, induction of IgG$_4$ and IgE switching, induction of germ line IgE heavy chain ε transcription, CD23 expression in normal human B cells, and induction of B cell proliferation in the presence of CD40L or anti-CD40 mAb.

IL-13 shares some functional properties with the cytokine IL-4. While many reported activities of IL-13 are similar to those of IL-4, IL-13 does not have growth promoting effects on activated T cells or T cell clones.

IL-13 exhibits certain biological activities by interacting with an IL-13 receptor ("IL-13R") on the surface of target cells. IL-13R and the IL-4 receptor ("IL-4R") share a common component, which is required for receptor activation. However, IL-13 does not bind to cells transfected with the IL-4 receptor.

**Summary of the Invention**

The invention is based in part on the discovery that administration of an inhibitor of IL-13 to a subject, or disrupting NK-T cell function in a subject, inhibits recurrence of the tumor. Accordingly, included in the invention is a use of an IL-13 inhibitor for the manufacture of a medicament for inhibiting the growth of a tumor in a subject wherein said tumor is selected from breast cancer, lung cancer and lymphoma.

The IL-13 inhibitor is preferably a non-toxic inhibitor. For example, the inhibitor preferably lacks a cytotoxic moiety such as a Pseudomonas exotoxin, ricin, abrin and Diphtheria toxin.

In some embodiments, the inhibitor is a ligand for an IL-13 polypeptide. One example of a suitable IL-13 ligand is a polypeptide that includes an IL-13 binding region of an IL-13 receptor.

Preferably, the polypeptide also includes an immunoglobulin polypeptide. In some embodiments, the inhibitor is a fusion protein that includes a soluble mammalian (*e.g.*, mouse or human) IL-13 receptor portion and a region of a human IgG$_1$ polypeptide. A preferred IL-13 ligand is sIL-13Rα2.Fc, which is described in Donaldson et al., J. Immunol. 161:2317-24, 1998.

Another suitable inhibitor is an IL-13 antagonist, *e.g.*, an antagonistic antibody that binds to an IL-13 polypeptide. The antibody can be either a polyclonal antibody or a monoclonal antibody. Antibodies to IL-13 can be made using techniques known in the art. For example, an IL-13 polypeptide may be used to immunize animals to obtain polyclonal and monoclonal antibodies which specifically react with the IL-13 protein, and which may inhibit binding of IL-13, or fragments thereof, to the receptor. Such antibodies may be obtained using the entire IL-13 as an immunogen, or by using fragments of IL-13, such as the soluble mature IL-13. Smaller fragments of IL-13 may also be used to immunize animals. The peptide immunogens additionally may contain a cysteine residue at the carboxyl terminus, and are conjugated to a hapten such as keyhole limpet hemocyanin (KLH). Additional peptide immunogens may be generated by replacing tyrosine residues with sulfated tyrosine residues. Methods for synthesizing such peptides are known in the art, for example, as described in Merrifield, J. Amer. Chem. Soc. 85, 2149-2154, 1963.

Tumor growth or occurrence is also inhibited by blocking the activity of a CD1-restricted NK-T cell. Preferably, the inhibitor binds to a CD1 molecule. Examples of CD-1 binding inhibitors include anti CD-1 antibodies or other soluble anti-CD1 ligands. Anti-CD 1 antibodies are known in art and are described in, *e.g.*, Roark et al., J. Imm. 160:3121-27, 1998.

In other embodiments, the inhibitor binds to an NK-T cell specific marker. An example of an NK-T cell specific marker is a Vα24 polypeptide. An example of this inhibitor is an antibody to a Vα24 polypeptide. The antibody can be either a monoclonal antibody or a polyclonal antibody and can be made using the methods described above. Antibodies that recognize Vα24 polypeptide are described in, *e.g.*, Prussin et al., J. Immunol.159, 5862-70, 1997.

The inhibitor can be administered to inhibit the growth of either a primary tumor, a metastatic tumor, or both. Thus, in some embodiments, the inhibitor is administered prior to detection of a primary tumor in the subject, *e.g.*, to inhibit the growth of a primary tumor. In other embodiments, the inhibitor is administered after detection of a primary tumor in the subject, *e.g.*, to inhibit the recurrence of a primary tumor or to inhibit metastasis.

In preferred embodiments, the inhibitor is administered in an amount sufficient to inhibit the growth of a tumor in the subject. For example, the inhibitor can be administered in a therapeutically effective amount. By "therapeutically effective amount" is meant the total amount of each active component of the pharmaceutical composition or method that is sufficient to show a meaningful patient benefit, *e.g.*, amelioration of symptoms of, healing of, or a decrease in size or other property of a tumor.

The subject can be, *e.g.,* a mammal such as a human, dog, cat, horse, cow, pig, or rodent (*e.g.,* mouse or rat).

The subject may be at an increased risk for developing a tumor. By "increased risk" is meant the subject is more likely to develop tumor than an age- matched control differing in one or more genetic factors or environmental factors, or both. For example, the subject may have an allele of a gene that predisposes the subject to increased risk of a tumor. The subject may have inherited the allele, *i.e.,* the subject may have a familial history of increased risk for developing a tumor. The alleles may be found in protooncogenes or tumor suppressor genes of the subject. Examples of protooncogenes include, *e.g.,* ras genes and myc genes. Examples of tumor suppressor genes include, *e.g.,* a retinoblastoma gene, a p53 gene, a BRCA gene, an APC, and a DCC gene. Environmental conditions can include those that expose the subject to known or suspected carcinogens.

Preferably, the tumor to be treated is a recurring tumor, *i.e.,* a tumor that is subject to immunosurveillance. Examples of suitable tumor types include carcinomas, sarcomas, or cancers of the blood cells. Thus, the tumor can be a breast, lung, tumor and a lymphoma (such as Burkitt's lymphoma).

If desired, the inhibitor can be administered along with an additional treatment regimen or regimens that are intended to inhibit tumor growth. For example, the additional treatment regimen can include administration of a second agent that inhibits tumor growth. A suitable second agent is one that increases levels of interferon gamma in the subject. The second agent can be interferon gamma itself.

The inhibitor and second agent (if desired) can be provided in the form of a pharmaceutical composition. Any suitable administration route and dosage schedule can be used to deliver the inhibitor, or a pharmaceutical composition that includes the inhibitor. When the inhibitor is administered orally, the inhibitor can be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95% inhibitor, and preferably from about 25 to 90% inhibitor. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of inhibitor, and preferably from about 1 to 50% inhibitor.

When a therapeutically effective amount of inhibitor is administered by intravenous, cutaneous or subcutaneous injection, the inhibitor will be in the form of a pyrogen-free, parenterally acceptable aqueous solution. The preparation of such parenterally acceptable protein solutions, having due regard to pH, isotonicity, stability, and the like, is within the skill in the art. A preferred pharmaceutical composition for intravenous, cutaneous, or subcutaneous injection should contain, in addition inhibitor, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art.

The amount of the inhibitor in the pharmaceutical composition of the present invention will depend upon the nature and severity of the condition being treated, and on the nature of prior treatments which the patient has undergone. Ultimately, the attending physician will decide the amount of inhibitor with which to treat each individual patient. Initially, the attending physician will administer low doses of inhibitor and observe the patient's response. Larger doses of inhibitor may be administered until the optimal therapeutic effect is obtained for the patient, and at that point the dosage is not generally increased further. It is contemplated that the various pharmaceutical compositions used to practice the method of the present invention should contain about 0.1 $\mu$g to about 100 mg of inhibitor per kg body weight. Preferred ranges include is a dosages of about 100$\mu$g to 6 mg, and 20 $\mu$g to 500 $\mu$g per kg of body weight.

The duration of intravenous therapy using the pharmaceutical composition of the present invention will vary, depending on the severity of the disease being treated and the condition and potential idiosyncratic response of each individual patient.

Ultimately the attending physician will decide on the appropriate duration of intravenous therapy using the pharmaceutical composition of the present invention. In some embodiments, the inhibitor is provided in a delayed-release composition. The timed-release composition can include a microparticle.

Microparticles are known in the art and include those described in, *e.g.,* US Patent No.6,013,258. Suitable material to include in microparticles includes polymeric material such as poly-lactic-co-glycolic acid (PLGA).

If desired the inhibitor can be administered along with a second or additional agents that enhance an immune response. Additional agents include, *e.g.,* adjuvants, cytokines (such as IL-12), and GM-CSF.

Other standard delivery methods, *e.g.,* biolistic transfer, or ex vivo treatment, can also be used. In ex vivo treatment, *e.g.,* antigen presenting cells (APCs), dendritic cells, peripheral blood mononuclear cells, or bone marrow cells, can be obtained from a patient or an appropriate donor and activated ex vivo with the immunogenic compositions, and then returned to the patient.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

Other features and advantages of the invention will be apparent from the following detailed description and claims.

**Brief Description of the Drawings**

FIGS. 1A and 1B are graphs showing the development of tumors in mice treated with anti-CD4 at different time periods.

FIGS. 2A and 2B are graphs showing the development of tumors in IL-4 KO, IL-4R, and STAT6 KO mice.

FIGS. 3A-3C are graphs showing cytokine production of T cells from 5-12RM-injected BALB/c mice with anti-CD3 stimulation.

FIGS. 4A and 4B are graphs showing the effect of IL-13 inhibitor on tumor recurrence.

FIG. 5A is a representation of an electrophoretogram showing expression of IL-13 and IL-4 mRNA in CD4$^+$ cells.

FIGS. 5B and 5C are histograms showing production of IL-13 and IL-4 by CD4$^+$NK1.1$^+$ and CD4$^+$NK1.1$^-$ T cells.

FIGS. 6A-6C are histograms showing cytokine production of CD4$^+$ T cells from 15-12RM-injected wild-type BALB/c mice and CD1 KO mice with anti-CD3 stimulation.

FIG. 7 is a graph showing the development of tumors in CD1 KO mice.

FIGS. 8A-8C are graphs showing CTL activity of CD1 KO mice injected with 15-12RM.

FIG. 9 is a graph showing CTL response in cells exposed to an HIV peptide vaccine, GM-CSF, and either an IL-13 inhibitor or a control peptide.

Detailed Description of the Invention

The invention provides compositions for inhibiting tumor growth in a subject.

Administration of a soluble form of an IL-13 receptor was found to inhibit tumor recurrence in a murine model system. Inhibition of NK-T cell activity has similarly been found to inhibit tumor recurrence in the model system. These results suggest that ligands for IL-13 polypeptide and NK-T cells act to inhibit immune responses, such as those involved in tumor surveillance. Immune responses were enhanced by inhibiting IL-13 function and/or NK-T cell function.

The compositions can be used to inhibit either primary tumor growth or secondary tumor growth (metastasis) in the subject. For example, the compositions,are suitable for use with a subject that has been diagnosed with a primary tumor in order to prevent additional growth of the primary tumor. Alternatively, or in addition, the compositions disclosed herein can also be used to inhibit the development of metastases of the primary tumor.

The invention will be further illustrated in the following examples,

**Example 1. Identification of IL-13 inhibitor-mediated inhibition of tumor recurrence**

This example describes studies identifying IL-13 as an important inhibitor of tumor immunosurveilance. Studies are first presented showing that treatment of mice with antibodies to CD4 molecules inhibits cytotoxic T-cell (CTL) mediated tumor surveillance if administered within 10 days of injection of tumor cells. Next are presented studies demonstrating that that tumor recurrence did occur in IL-4 KO mice, but not in IL-4R KO mice or signal transducer and activator of transcription (STAT)6 KO mice. These results suggest IL-13 is involved in tumor recurrence. These results are substantiated by showing that CD-4 depleted mice produced lower levels of IL-13, as well as data showing that administration of (sIL-13Ra2.Fc), an IL-13 inhibitor, inhibits tumor recurrence.

For the studies described herein, female BALB/c mice were purchased from Charles River Breeding Laboratories (Frederick, MD). IL-4 knockout (KO) mice (Noben-Trauth et al., Transgenic Res 5: 487-91, 1996) on a BALB/c background were obtained from the Jackson Laboratory (Bar Harbor, ME). The BALB/c mouse strain congenic for the C57BL/6 NK1.1 locus was provided by Drs. Anthony Scalzo and Wayne Yokayama (Scalzo, et al., Immunogenetics 41: 148-51, 1995). C57BL/6 mice, IL-4R KO (Noben-Trauth et al., Proc. Natl Acad. Sci. U S A 94: 10838-43,1997), signal transducer and activator of transcription (STAT)6 KO (Kaplan et al., Immunity 4: 313-9, 1996) and CD1 KO (Smiley et al., Science 275: 977-9, 1997) mice having the BALB/c background were bred under pathogen-free conditions. All the mice were maintained in a pathogen-free animal facility and were used at 6-10 weeks of age. Animal experiments were all approved by the National Cancer Institute (NCI) Animal Care and Use Committee.

15-12RM tumor cells were made by transfecting BALB/c 3T3 fibroblasts first with HIV-1 IIIB gp160 and then with mutant ras and myc genes to make them tumorigenic (Matsui et al., J. Immunol. 163: 184-193, 1999). This and control BALB/c 3T3 cells transfected with Neo only (18Neo) (Takahashi et al., Proc. Natl. Acad. Sci. USA 85: 3105-09,1988) were maintained in complete T cell medium (CTM) which consisted of RPMI1640 with 10% FCS, L-glutamine, sodium pyruvate, nonessential amino acids, penicillin, streptomycin and $5 \times 10^{-5}$ M 2-mercaptoethanol, containing geneticin (200 $\mu$g/ml) (Sigma, St. Louis, MO).

Purified rat anti-mouse CD4 mAb (GK1.5 (Wilde et al., J. Immunol. 131: 2178-2183, 1983)) was obtained from the Frederick Cancer Research and Development Center, NCI (Frederick, MD). IL-13 inhibitor, a fusion protein of murine IL-13R$\alpha$2 and human IgG1 (sIL-13R$\alpha$2.Fc), was made as described previously (Donaldson et al., J Immunol 161: 2317-24, 1998) and provided from the Research Support Team at Genetics Institute, Cambridge, MA. Anti-CD3 (2C11 (Leo et al., Proc. Natl Acad. Sci. USA 84: 1374-78, 1987)), anti-CD28 (37.51), FITC-conjugated anti-CD4 (GK1.5) and PE-conjugated anti-NK1.1 (PK136) were obtained from Pharmingen (San Diego, CA). Anti-Ia, anti-CD11b, anti-CD11c, anti-DX5, anti-CD4 and anti-CD8 magnetic beads were purchased from Miltenyi Biotec Inc (Auburn, CA). Recombinant mouse IL-2 was obtained from Pharmingen. Trizol, SuperScript cDNA synthesis kit, and PCR SuperMixture were purchased from Life Technologies (Rockville, MD).

One million 15-12RM cells in 200 $\mu$l of PBS were injected subcutaneously on the right flank of the mouse. To deplete CD4$^+$ cells *in vivo*, mice were inoculated intraperitoneally with 0.2 ml of PBS containing 0.5 mg of anti-CD4 or control rat IgG (Sigma, St. Louis, MO) as indicated. In some experiments, mice were treated every other day for 8 days with 0.2 mg of IL-13 inhibitor (sIL-13R$\alpha$2.Fc) by intraperitoneal injection in 0.1 ml PBS.

For in vitro activation of T cells, CD4$^+$ T cells and CD4$^+$ NK-T cells, 96 well plates were coated with anti-CD3 (10 $\mu$g/ml) over night at 4 °C. The plates were washed three times with PBS before use. Single-cell suspensions of splenocytes from 15-12RM-injected mice were prepared at different time points after the injection. T cells were negatively selected from splenocytes by using magnetic beads coated with antibodies for Ia, CD11b, CD11c and DX5. CD4$^+$ T cells were obtained by depleting CD8$^+$ cells from T cells by using anti-CD8 magnetic beads. The cells were cultured at a density of $1 \times 10^5$/well of a 96 well plate in 200 $\mu$l CTM. Two days after the stimulation 100 $\mu$l of culture medium was harvested from each well and stored at -70°C until cytokine measurement. In some experiments, CD4$^+$ T cells ($1 \times 10^6$/well) were stimulated with CD1-transfected L cells (Chen et al., J Immunol 159: 2240-9, 1997) or L cells ($2 \times 10^4$/well) with anti-CD28 (10 $\mu$g/ml) and IL-2 (20 units/ml). After one week, culture medium was harvested and stored at -70 °C until cytokine measurement.

Spleen cells of C57BL/6 mice or BALB/c mice congenic for the NK1.1 locus were isolated by passing through a CD4-enrichment column (Cedar Lane, Westbury, NY) and then sorted for CD4$^+$ NK1.1$^+$ or CD4$^+$ NK1.1$^-$ T cells by using FACStar (Becton Dickenson, Mountain View, CA). To detect cytokine mRNA, the cells were cultured at a density of $1 \times 10^5$/well of a 96 well plate in 200 $\mu$l CTM. Eight hours after the stimulation cells were harvested from each well and stored at -70 °C until RNA extraction. For cytokine measurement, $2 \times 10^4$ purified cells were stimulated in 96 well plates coated with 10 $\mu$g/ml each of anti-CD3 and anti-CD28 mAbs. Supernatants were collected at 48 hours and assayed for IL-4, IL-13 and IFN-$\gamma$.

The concentration of IL-4, IL-13 (R&D, Minneapolis, MA or Endogen, Woburn, MA), IFN-$\gamma$ (R&D) in the culture supernatant were determined by ELISA kit according to the manufacturer's instructions. All samples were assayed in triplicate.

Cytotoxic activity of CD8$^+$ T cells against several target cells was measured by a 4 h-$^{51}$Cr release assay. CD8$^+$ T cells were purified from splenocytes by depletion of B cells, macrophages, DC, NK cells and CD4$^+$ cells using magnetic beads (Milteny Biotec Inc.). The percentage of specific $^{51}$Cr release was calculated as follows:

$$100 \times (\text{experimental release} - \text{spontaneous release})/(\text{maximum release} - \text{spontaneous release}).$$

Maximum release was determined from supernatants of cells that were lysed by addition of 5 % Triton X-100. Spontaneous release was determined from target cells incubated without added effector cells.

Total RNA was extracted from sorted CD4$^+$ NK-T cells with Trizol reagent. cDNAs were synthesized by using the Superscript cDNA synthesis kit according to manufacturer's instructions. PCR was done with Supermixture containing 0.2 $\mu$M primers by the GeneAmp 9700 PCR system (Perkin-Elmer, Norwalk, CT). The PCR was run for 28 cycles of 94 °C for 30 s, 55 °C for 1 min, 74°C for 1 min. The primers for IL-4 were purchased from Promega (Madison, WI). The sequence of the primers used were follows: IL-13 (sense) 5'-GACCCAGAGGATATTGCATG-3' (SEQ ID NO:1); IL-13 (antisense) 5'-CCAGCAAAGTCTGATGTGAG-3' (SEQ ID NO:2); HPRT (sense) 5'-GTTGGATACAGGCCAGACTTTGTTG-3' (SEQ ID NO:3); HPRT (antisense) 5'-TCGGTATCCGGTCGGATGGGAG-3' (SEQ ID NO:4).

The data were analyzed for statistical significance using a Log-Rank test. The data were considered significant at $P<0.05$.

*Timing of anti-CD4 treatment to affect tumor recurrence*

The effect of the timing of anti-CD4 treatment relative to tumor recurrence was examined in the 15-12RM mouse model.

15-12RM tumor cells were injected subcutaneously (s.c.) in the right flank of different groups of BALB/c mice at day 0. Anti-CD4 was administered to the different mice in the groups for various times prior to or following implantation. The percentage of mice developing tumors following administration of anti-CD4 was examined.

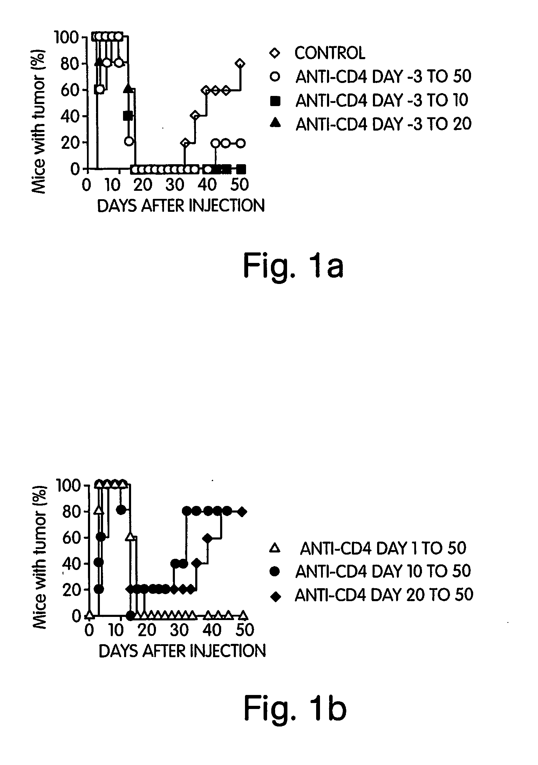

The results are presented in FIGS. 1A and 1B. FIG. 1A shows tumor growth in groups of mice in which anti-CD4 was not administered (control), administered at day -3 to 50, day -3 to 30, or day -3 to 10. FIG. 1B shows tumor growth in mice for which anti-CD4 was administered at day 1 to day 50, day 10 to day 50, and day 20 to day 50.

The mice of each group were intraperitoneally inoculated with 0.5mg of anti-CD4 mAb (GK1.5) during the indicated periods after 15-12RM injection. The antibody was inoculated every day for the first 3 days and then twice a week. Five mice were used for each group. $P<0.02$ by Log-Rank test between control and anti-CD4 day -3 to 10, 20 or day 1 to 50. Similar results were obtained in another experiment.

Tumors were detectable within 5 days (Matsui et al., J. Immunol. 163: 184-193, 1999) (FIG. 1A, control group). The tumors regressed and disappeared after 10-15 days. However, recurrence of the tumors was observed between 20 and 40 days after inoculation and recurrent tumors did not regress (FIG. 1A). Tumor recurrence observed in control mice occurred because of incomplete elimination of tumor cells by $CD8^+$ CTL during the tumor regression phase. Conversely, in anti-CD4-treated mice, tumor cells were completely eliminated after the initial growth and did not recur (FIG. 1A and Matsui et al., J. Immunol. 163: 184-193, 1999). These results suggested that $CD4^+$ T cells negatively regulated immunosurveillance against tumor cells by CTL.

To examine when $CD4^+$ T cells regulate CTL specific for tumor cells, mice were treated with CD4 antibody over different intervals after 15-12RM injection (Fig. 1A). All mice in the groups which started to receive CD4 antibody 3 days before 15-12RM injection and stopped receiving antibody at day 10 or day 20 were resistant to tumor recurrence in a similar fashion to the mice treated from day-3 to the end of the experiment. Although initiation of anti-CD4 treatment could be delayed until day 1, starting treatment on day 10 was too late to have any impact on the late recurrence (FIG. 1B). This suggests that immunosurveillance of CTL against tumor cells is regulated by $CD4^+$ T cells at an early tumor growth phase. Depletion of $CD4^+$ T cells during the first 10 days was both necessary and sufficient to prevent this regulation.

*Down-regulation of CTL immunosurveillance through IL-4R-STAT6*

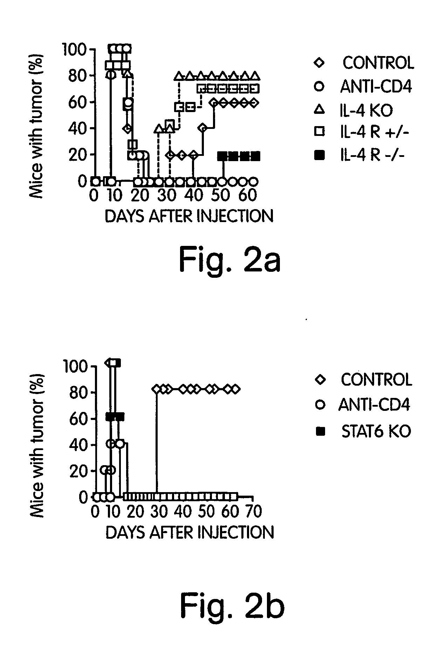

IL-4 KO and IL-4R KO mice were used to clarify whether IL-4 and its signaling pathway are involved in down-regulation of immunosurveillance by CTL. The results are shown in FIGS. 2A and 2B. FIG. 2A shows the results obtained in control mice or mice treated with anti-CD4 antibody or BALB/c background IL-4 KO, IL-4R KO, or heterozygote littermate mice were injected subcutaneously with $1 \times 10^6$ 15-12RM cells. The mice in the anti-CD4-treated group were treated with anti-CD4 mAb every day from 3 days before 15-12RM injection until the day of the injection and twice a week thereafter. Five mice were used for each group except for the IL-4R +/- group in which 7 mice were used. $P<0.05$ by Log-Rank test between the control group and the IL-4R-/- group. The result shown is a representative of two experiments with similar results.

For the results shown in FIG. 2B, BALB/c-STAT6 KO mice or wild-type BALB/c mice either untreated or treated with anti-CD4 were injected subcutaneously with $1 \times 10^6$ 15-12RM cells. The mice in anti-CD4-treated group were treated with anti-CD4 mAb every day from 3 days before 15-12RM injection and twice a week thereafter. Five mice were used for each group. $P<0.02$ by Log-Rank test between the control group and the STAT6 KO group. One representative experiment of three is shown.

Tumor recurrence occurred in IL-4 KO mice; indeed, the tumor growth pattern was not significantly different from that of control mice (FIG. 2A and Matsui et al., J. Immunol. 163: 184-193, 1999). In contrast, in IL-4R KO mice, tumors did not recur after the initial growth and regression, although in heterozygous littermates they recurred as in wild-type mice (FIG. 2A).

STAT6 activation is known to be necessary for signaling through the IL-4R signal pathway (Shimoda et al., Nature 380: 630-3 1996; Takeda et al., Nature 380: 627-30, 1996; and Kaplan et al., Immunity 4: 313-9, 1996). Accordingly, to investigate whether the protection observed in IL-4R KO mice but not in IL-4 KO mice was dependent on the IL-4R signal pathway, STAT6 KO mice were inoculated with 15-12RM (FIG. 2b). The STAT6 KO mice were as resistant to tumor recurrence as CD4-depleted mice. This suggested that immunosurveillance by CTL was limited through the IL-4R-STAT6 signaling pathway, but this regulation did not require IL-4 itself. Therefore, IL-13, the only other cytokine known to signal through the IL-4R-STAT6 signaling pathway, is likely to play a significant role in this limitation.

*Production of cytokines in CD4-depleted mice*

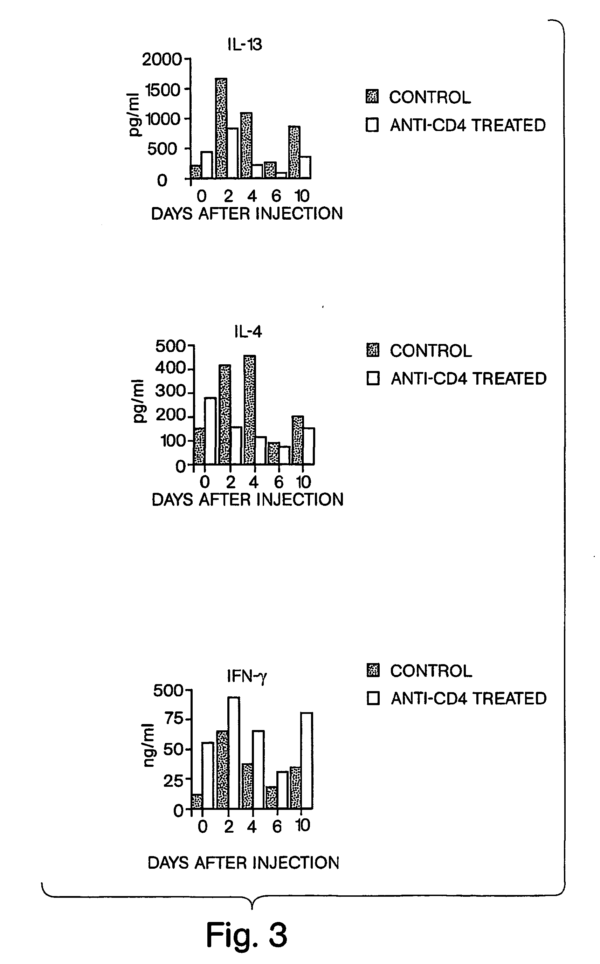

Because T cells are major producers of IL-13 and IL-4, *in vitro* production of both cytokines by T cells from 15-12RM-injected mice was measured by ELISA after anti-CD3 stimulation. The results are shown in FIG. 3. On days 0,2,4,6,10 after injection of 15-12RM cells, two mice per group were sacrificed and their spleen cells were harvested. A fraction of the cells was used to purify T cells. One hundred thousand freshly isolated T cells from 15-12RM-injected mice were cultured in an anti-CD3 coated plate for 48 h. The culture supernatant was collected and concentration of each cytokine was determined by ELISA. The figure shows the average value of two mice. Similar results were obtained in an additional experiment.

Both IL-13 and IL-4 production in T cells from control mice are elevated as early as 2 days after 15-12RM injection. The IL-13 and IL-4 production reached a maximum on days 2 and 4, respectively, and then dropped to baseline by day 6 after injection. In the T cells from CD4-depleted mice, the absolute amount of IL-13 was 50-75 % less than that of the cells from 15-12RM injected control mice, even though production of IL-13 was up-regulated on day 2. Additionally, IL-4 production was not up-regulated even after 15-12RM injection. Thus, splenic T cells from CD4-depleted mice, which are resistant to tumor recurrence, produced less IL-13 and IL-4.

Production of IFN-γ, which plays a protective role against tumor growth in this system (Matsui et al., J. Immunol. 163: 184-193, 1999), was measured. In contrast to IL-13 and IL-4 production, T cells from anti-CD4-treated mice produced 25-100 % more IFN-γ than the cells from control mice. These results suggested that removing CD4[+] T cells suppresses production of IL-13 and IL-4 but allows more IFN-γ production, which was shown previously (Matsui et al., J. Immunol. 163: 184-193, 1999) to be necessary for the immunosurveillance.

*An IL-13 inhibitor (sIL-13Pα2.Fc) inhibits tumor recurrence*

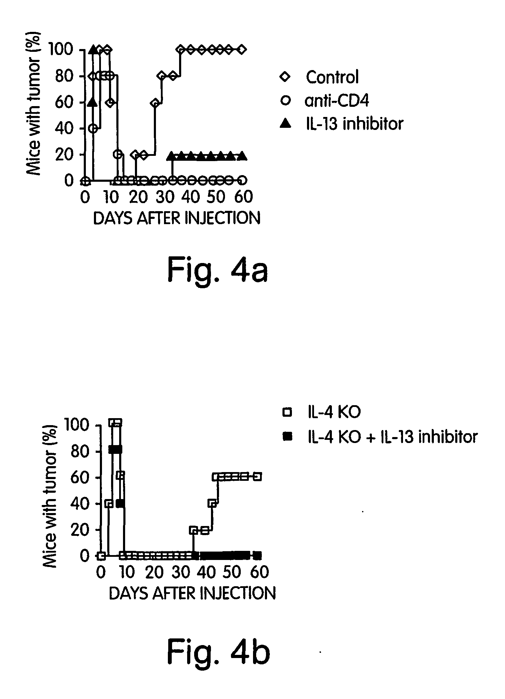

The results obtained above were compatible with either IL-13 alone being necessary for down-regulation of immunosurveillance or the possibility that either IL-4 or IL-13 was sufficient and eliminating both would be necessary to prevent recurrence. To distinguish these possibilities, the effect of IL-13 inhibitor (sIL-13Rα2.Fc) in both wild-type and IL-4 KO mice was tested. The results are shown in FIGS. 4A and 4B. Control BALB/c mice, mice treated with either anti-CD4 or IL-13 inhibitor (sIL-13Rα2.Fc) (FIG. 4A) or BALB/c-IL-4 KO mice either untreated or treated with IL-13 inhibitor (FIG. 4B) were injected subcutaneously with $1 \times 10^6$ 15-12RM cells. The anti-CD4 (0.5 mg) was inoculated on day 0, 1, 2, 6 and 10 after 15-12RM. The mice in IL-13 inhibitor-treated groups were treated with sIL-13Rα2.Fc (0.2 mg) every other day from day 0 to day 8. Five mice were used for each group. *P*<0.01 by Log-Rank test between the control group and the IL-13 inhibitor group. *P*<0.02 by Log-Rank test between the IL-4 KO group and the IL-4KO + IL-13 inhibitor group. One representative experiment of two is shown.

The IL-13 inhibitor was inoculated every other day from day 0 to day 8 after 15-12RM injection, but did not prevent the initial growth and regression of the tumor. However, when IL-4 KO mice, which are not resistant to tumor recurrence, were treated with IL-13 inhibitor, they were completely protected from the recurrence. When wild-type mice were treated with the IL-13 inhibitor, they also behaved like CD4-depleted mice and IL-13 inhibitor-treated IL-4 KO mice. These results showed that in this biphasic tumor model, IL-13 is necessary for the tumor recurrence, regardless of the capacity of the mice to make IL-4.

## Example 2. Inhibition of tumor growth by disrupting NK-T cell function

This example presents data showing that IL-13 is produced by NK-T cells, and that CD 1 KO mice, which lack NK-T cells, produce low levels of IL-13. Also illustrated in this example is inhibition of tumor growth in CD1 KO mice.

*NKT cells can produce IL-13*

Treatment during the first 8 days with IL-13 inhibitor protected mice from tumor recurrence (FIGS. 4A and 4B). CD4[+]NK1.1[+] T cells are among the earliest cells to produce a large amount of IL-4 upon TCR activation (Chen et al., J Immunol 159: 2240-9, 1997).

Accordingly, the ability of CD4[+] NKT cells to produce IL-13 was examined. Since BALB/c mice do not express the NK1.1 allele recognized by available mAbs, CD4[+] NKT cells from C57BL/6 mice or BALB/c mice congenic for the NK1.1 locus were sorted by purifying CD4[+] T cells and sorting for CD4[+]NK1.1[+] and CD4[+]NK1.1[-] populations, and stimulated these with anti-CD3 antibody.

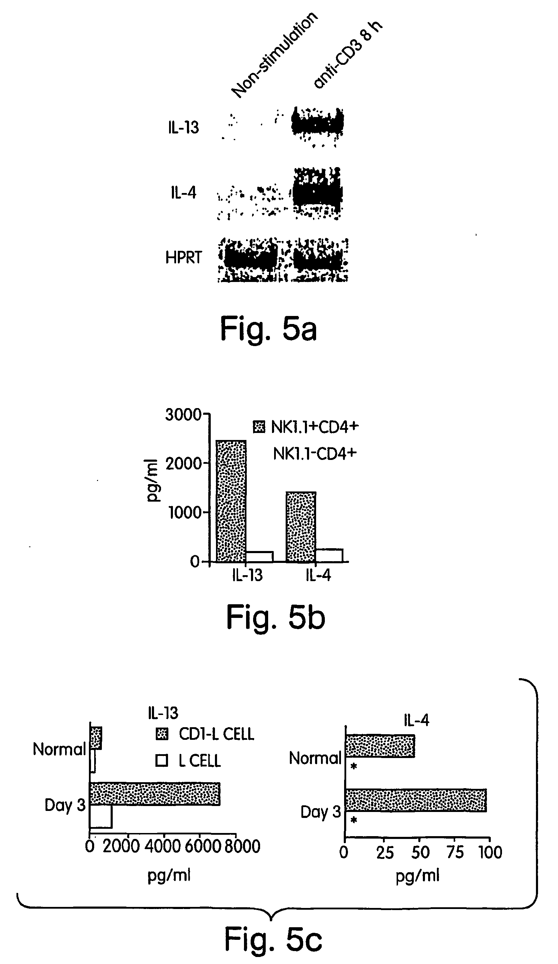

Production of IL-13 mRNA and protein was measured. The results are shown in FIGS. 5A-5C. Freshly sorted splenic CD4[+] NK-T cells from C57BL/6 mice were stimulated with plate-bound anti-CD3 (2C11) for 8 h. The cells were harvested and mRNA for IL-13 and IL-4 was detected by RT-PCR. FIG. 5A shows expression of mRNA of IL-13 and IL-4 in CD4[+] NK-T cells after anti-CD3 stimulation.

FIG. 5B demonstrates production of IL-13 and IL-4 by CD4+NK1.1+ and CD4+NK1.1- T cells. Freshly sorted splenic CD4+NK1.1+ and CD4+NK1.1- T cells from BALB/c mice congenic for the NK1.1 locus were stimulated with plate-bound anti-CD3 (2C11) and anti-CD28 (37.51) for 48 h. Supernatants were collected at 48 h and assayed for IL-4 and IL-13 by ELISA.

Production of IL-13 and IL-4 by CD4+ T cells from 15-12RM-injected mice stimulated with CD1-transfected L cells is shown in FIG. 5C. CD4+ T cells purified from spleen cells from normal and 15-12RM-injected (on day 3 after injection) BALB/c mice were stimulated with CD1-transfected L cells or non-transfected L cells for a week. Anti-CD28 mAb (10 μg/ml) and IL-2 (20 units/ml) were also added in the culture. The culture supernatant was collected and concentration of IL-13 and IL-4 were determined by ELISA. Asterisks indicate a level under the detection limit (<15.6 pg/ml).

IL-13 was clearly induced in CD4+NK1.1+ T cells by anti-CD3 stimulation, whereas CD4+NK1.1- T cells made little (FIGS. 5A and 5B). Furthermore, when CD4+ T cells from 15-12RM-injected BALB/c mice were stimulated with CD1-transfected L cells, they produced more IL-13 and IL-4 than the cells from non-tumor-bearing mice (FIG. 5C) but did not produce detectable IFN-γ (data not shown). The lower level of IL-4 in FIG. 5C than in Fig. 5B may be due to consumption during the 7-day culture in the former, *versus* 48 h in the latter. In contrast, because the purified CD4+ T cells do not have IL-13 receptors, that cytokine should not have been consumed. Thus, CD4+ NKT cells can indeed make IL-13, and are a primary source of this cytokine. Moreover, this activity appears to be enhanced in tumor-bearing animals.

*IL-13 production is markedly decreased in CD1 KO mice*

If NKT cells are a major source of IL-13, then their absence in CD1 KO mice should markedly reduce IL-13 production. Accordingly, the *in vitro* production of IL-13, IL-4 and IFN-γ in CD4+ T cells of 15-12RM-injected CD1 KO mice was examined. These mice lack CD1-restricted NKT cells, including CD4+ NKT cells, but retain conventional MHC class II-restricted CD4+ T cells.

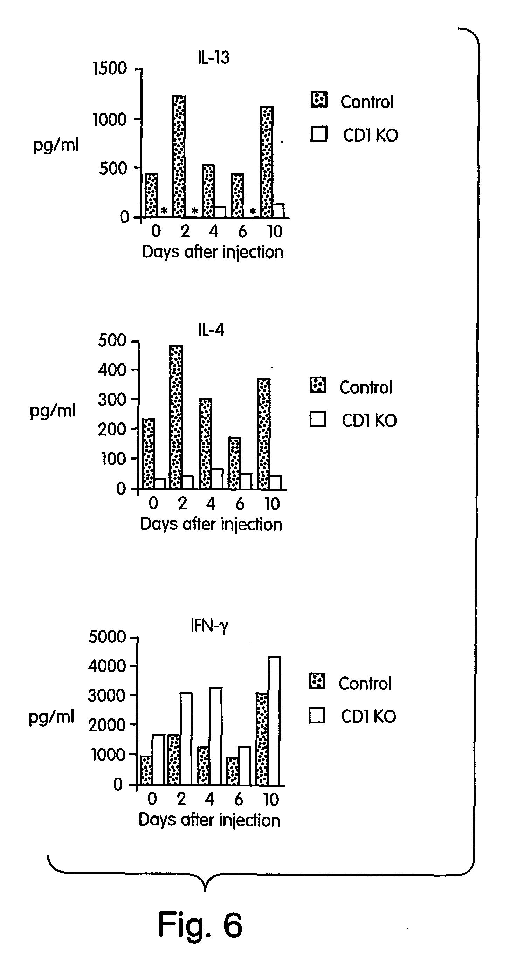

The results are shown in FIG 6. Two mice per group were sacrificed and their spleen cells were harvested on days 0, 2, 4, 6, 10 after injection of 15-12RM cells. A fraction of the cells was used to purify CD4+ T cells. One hundred thousand freshly isolated T cells from 15-12RM-injected mice were cultured in an anti-CD3 coated plate for 48 h. The culture supernatant was collected and concentration of each cytokines was determined by ELISA. The each value shows the average of two mice. The results are representative of two independent experiments. Asterisks indicate a level under the detection limit (<7.8 pg/ml).

CD4+ T cells purified from spleen cells of CD1 KO mice produced only a slight amount of IL-13 and IL-4 after 15-12RM injection, whereas CD4+ T cells from wild-type mice up-regulated the production of these cytokines on days 2, 4 and 10 after tumor inoculation. This indicates that the majority of IL-13 production was from or dependent on NKT cells. Conversely, the IFN-γ produced was even higher in the cells of CD1 KO mice than in those of wild-type mice.

*CD1 KO mice show low levels of tumor recurrence*

Because CD4+ NKT cells can produce IL-13 and indeed are responsible for much of the IL-13 production, and these cells would be depleted by anti-CD4 treatment in addition to conventional CD4+ T cells, it was hypothesized that NKT cells play a critical role in down-regulation of tumor immunosurveillance by CTL. To test this hypothesis, CD1 KO mice were inoculated with 15-12RM and the effect of tumor growth with or without and-CD4 treatment was measured.

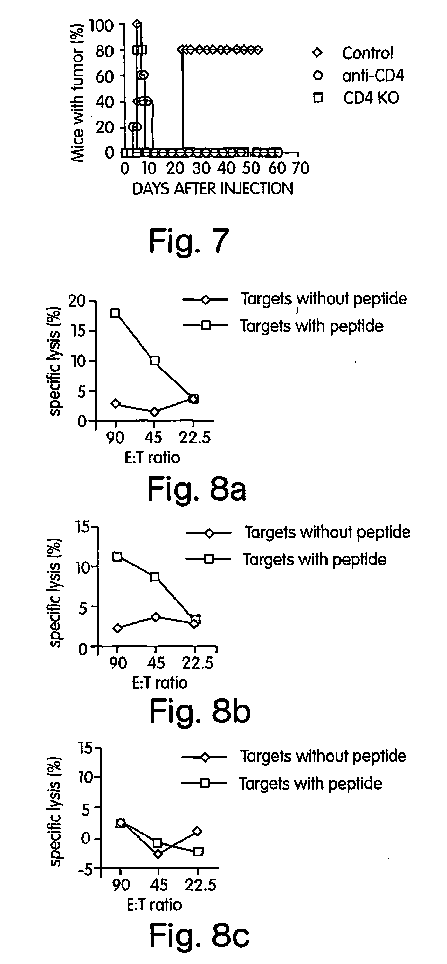

The results are shown in FIG. 7. BALB/c background CD1 KO mice and wild-type BALB/c mice either untreated or treated with anti-CD4 were injected subcutaneously with $1 \times 10^6$ 15-12RM cells. The mice in the anti-CD4-treated group were treated with anti-CD4 (0.5 mg) starting 3 days before 15-12RM injection and twice a week thereafter. Five mice were used for each group. *P*<0.02 by Log-Rank test between the control group and the CD1 KO group. One representative experiment of four is shown. In the CD1 KO mice, tumors grew initially and regressed in the same time period as in wild-type mice. However, CD1 KO mice were as resistant to tumor recurrence as anti-CD4-treated wild-type mice. Thus, CD1-restricted NKT cells appeared to be necessary for the down-regulation of immunosurveillance.

The activity of CD8+ T cells purified from splenocytes of anti-CD4-treated (FIG. 8A), CD1 KO (FIG. 8B) and control mice (FIG. 8C) examined for CTL activity at 12 days after 15-12RM injection against 18Neo targets pulsed with 1 μM of P18IIIB or unpulsed, without any *in vitro* stimulation. One representative experiment of three is shown in FIG. 8A-8C, respectively.

In CD4-depleted mice, protection against tumor recurrence appeared to be associated with higher CTL activity of CD8+ T cells immediately *ex vivo* without restimulation than in control mice (FIG. 8A and Matsui et al., J. Immunol. 163: 184-193, 1999). To determine whether the same applied to CD1 KO mice, the CTL activity of purified CD8+ T cells from 15-12RM-injected CD1 KO mice was compared with that of the CD8+ T cells from tumor injected control and anti-CD4-treated mice (FIG. 8B). Freshly isolated CD8+ T cells from CD1 KO (FIG. 8B) lysed P18IIIB-pulsed 18Neo at a low level without *in vitro* stimulation, whereas CD8$^+$ T cells from intact tumor-bearing mice did not kill P18IIIB-pulsed 18Neo without restimulation (FIG. 8C). Thus, the presence of NKT cells led to reduced CTL activity immediately *ex vivo.*

Taken together, these results demonstrate that IL-13 produced either by CD1-restricted CD4$^+$ NKT cells, or possibly by other cells dependent on NKT cells, at an early growth phase of the tumor (within 10 days) triggers negative regulation of CTL immunosurveillance, and that this negative regulation is mediated through the IL-4R$\alpha$-STAT6 pathway.

**Example 3. An IL-13 inhibitor enhances a CTL response to a synthetic peptide HIV vaccine**

The ability of an IL-13 inhibitor for enhancing a cytotoxic T cell lymphocyte (CTL) response was examined.

BALB/c mice were immunized subcutaneously with a synthetic peptide vaccine PCLUS6.1-18IIIB (20 nmoles) (US Patent No. 5,932,218) that was mixed with GM-CSF (5 ug) in an emulsion adjuvant, Montanide ISA-51 on day 0. On each of days 0,1,2,4, and 6, 200 ug of the IL-13 inhibitor IL-13Ra2-Fc was administered intraperitoneally. Controls received a control IgG instead of IL-13 inhibitor.

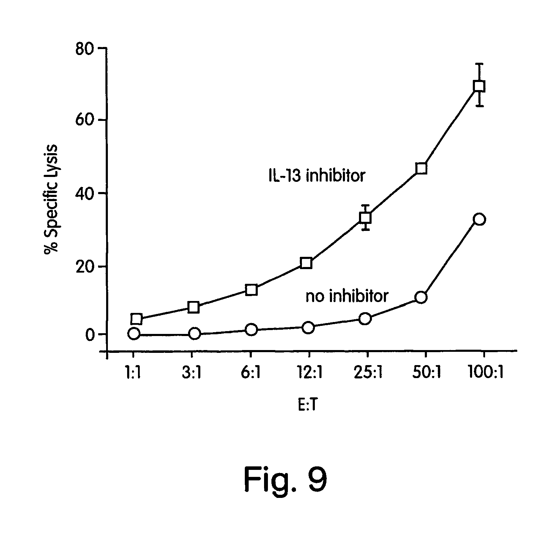

The subsequent CTL response in the spleens of the test and control mice, harvested 14 days later and stimulated for 1 week in culture, was measured and is plotted in the graph shown in FIG. 9. Targets cells were coated with P18IIIB peptide.

Significantly higher CTL-mediated lysis was observed when the IL-13 inhibitor was administered along with a synthetic peptide HIV vaccine to mice. These results demonstrate that an increased the cytotoxic T lymphocyte (CTL) response in the presence of the inhibitor as compared to the vaccine without the IL-13 inhibitor.

**Claims**

1. Use of an IL-13 inhibitor, wherein said inhibitor comprises an IL-13 binding region of an IL-13 receptor or is an antibody which binds to IL-13, for the preparation of a pharmaceutical composition for inhibiting the growth of a tumor in a subject, wherein said tumor is selected from the group consisting of a breast cancer, lung cancer, and lymphoma.

2. The use of claim 1, wherein said inhibitor is administered:

   a) in an amount sufficient to inhibit the growth of a tumor in said subject;
   b) prior to detection of a primary tumor in said subject;
   c) after detection of a primary tumor in said subject;
   d) prior to treatment of a tumor in said subject;
   e) following treatment of a tumor in said subject;
   f) within two weeks prior to surgical resection of a tumor in said subject;
   g) within two weeks after surgical resection of a tumor in said subject;
   h) systemically to said subject;
   i) topically to said subject;
   j) by a route selected from intravenous, subcutaneous, and intramuscular delivery; or
   k) in a delayed-release composition.

3. The use of claim 1, wherein said subject is at increased risk for developing said tumor or has an allele of a gene that increases the risk of developing said tumor in said subject.

4. The use of claim 3, wherein said gene is a tumor suppressor gene selected from the group consisting of a retinoblastoma gene, a p53 gene, a BRCA gene, a APC, and a DCC gene.

5. The use of claim 2, wherein said inhibitor is administered within two weeks after surgical resection of a tumor in said subject and wherein said inhibitor is administered concurrently with treatment of a tumor in said subject.

6. The use of claim 1, wherein said inhibitor further comprises an immunoglobulin polypeptide.

7. The use of claim 1, wherein said inhibitor is a fusion protein comprising a murine IL-13R$\alpha$2 polypeptide and an Fc region of a IgG$_1$ polypeptide, or a fusion protein comprising a human IL-13R$\alpha$2 polypeptide and an Fc region of a IgG$_1$ polypeptide.

8. The use of claim 1, wherein said antibody is a polyclonal antibody or a monoclonal antibody.

**9.** The use of claim 1, wherein said subject is a human.

**10.** The use of claim 1, further comprising administering to said subject a second agent that inhibits tumor growth, wherein said second agent increases interferon gamma levels in said subject.

**11.** The use of claim 2, wherein said delayed-release composition comprises a microparticle.

**12.** The use of claim 11, wherein said microparticle is poly-lactic-co-glycolic acid (PLGA).

**13.** Use of a non-cytotoxic inhibitor of IL-13 for the preparation of a pharmaceutical composition for inhibiting the growth of a tumor in a subject, wherein said inhibitor comprises an IL-13 binding region of an IL-13 receptor or is an antibody which binds to IL-13 and said tumor is selected from the group consisting of a breast cancer, lung cancer, and lymphoma.

**14.** The use of claim 13, wherein said inhibitor inhibits in said subject IL-13 gene expression or IL-13 activity.

**15.** The use of claim 13, wherein said inhibitor comprises an IL-13 binding region of an IL-13 receptor and further comprises an immunoglobulin polypeptide.

**16.** The use of claim 13, wherein said antibody is a polyclonal antibody or a monoclonal antibody.


**Patentansprüche**

**1.** Verwendung eines IL-13-Inhibitors, wobei genannter Inhibitor eine IL-13 bindende Region eines IL-13-Rezeptors umfasst oder ein Antikörper ist, der an IL-13 bindet, für die Herstellung einer pharmazeutischen Zusammensetzung zur Inhibition des Tumorwachstums in einem Individuum, wobei genannter Tumor aus der Gruppe ausgewählt wird, die aus Brustkrebs, Lungenkrebs und Lymphom besteht.

**2.** Die Verwendung nach Anspruch 1, wobei genannter Inhibitor wie folgt verabreicht wird:

  a) in einer Menge, die ausreicht, das Wachstum eines Tumors in genanntem Individuum zu inhibieren;
  b) vor dem Nachweis eines primären Tumors in genanntem Individuum;
  c) nach dem Nachweis eines primären Tumors in genanntem Individuum;
  d) vor der Behandlung eines Tumors in genanntem Individuum;
  e) anschliessend an die Behandlung eines Tumors in genanntem Individuum;
  f) innerhalb von zwei Wochen vor operativer Entfernung eines Tumors in genanntem Individuum;
  g) innerhalb von zwei Wochen nach operativer Entfernung eines Tumors in genanntem Individuum;
  h) systemisch an genanntes Individuum;
  i) äußerlich an genanntes Individuum;
  j) auf einem Weg, der ausgewählt ist aus intravenöser, subkutaner und intramuskulärer Verabreichung; oder
  k) in einer verzögert freisetzenden Zusammensetzung.

**3.** Die Verwendung nach Anspruch 1, wobei genanntes Individuum ein erhöhtes Risiko hat, genannten Tumor zu entwickeln, oder ein Allel eines Gens besitzt, das das Risiko, genannten Tumor zu entwickeln, in genanntem Individuum erhöht.

**4.** Die Verwendung nach Anspruch 3, wobei genanntes Gen ein Tumorsuppressorgen ist, das aus der Gruppe bestehend aus einem Retinoblastomgen, einem p53-Gen, einem BRCA-Gen, einem APC-Gen und einem DCC-Gen, ausgewählt wird.

**5.** Die Verwendung nach Anspruch 2, wobei genannter Inhibitor innerhalb von zwei Wochen nach operativer Entfernung eines Tumors in genanntem Individuum verabreicht wird, und wobei genannter Inhibitor zeitgleich mit einer Tumorbehandlung des genannten Individuums verabreicht wird.

**6.** Die Verwendung nach Anspruch 1, wobei genannter Inhibitor zusätzlich ein Immunglobulinpolypeptid umfaßt.

**7.** Die Verwendung nach Anspruch 1, wobei genannter Inhibitor ein Fusionsprotein ist, das ein murines IL-13Rα2-Polypeptid und eine Fc-Region eines IgG1-Polypeptids umfaßt, oder ein Fusionsprotein, das ein humanes IL-13Rα2-Polypeptid und eine Fc-Region eines IgG1-Polypeptids umfaßt.

8. Die Verwendung nach Anspruch 1, wobei genannter Antikörper ein polyklonaler Antikörper oder ein monoklonaler Antikörper ist.

9. Die Verwendung nach Anspruch 1, wobei genanntes Individuum ein Mensch ist.

10. Die Verwendung nach Anspruch 1, die zusätzlich die Verabreichung eines zweiten tumorinhibierenden Wirkstoffs an genanntes Individuum umfaßt, wobei genannter zweiter Wirkstoff die Interferon-gamma-Konzentration in genanntem Individuum anhebt.

11. Die Verwendung nach Anspruch 2, wobei genannte verzögert freisetzende Zusammensetzung einen Mikropartikel umfaßt.

12. Die Verwendung nach Anspruch 11, wobei genannter Mikropartikel Polylaktid-co-Glykolid (PLGA) ist.

13. Die Verwendung eines nicht zytotoxischen IL-13-Inhibitors zur Herstellung einer pharmazeutischen Zusammensetzung zur Inhibition des Tumorwachstum in einem Individuum, wobei genannter Inhibitor eine IL-13 bindende Region eines IL-13-Rezeptors umfaßt, oder ein Antikörper ist, der an IL-13 bindet und genannter Tumor aus der Gruppe ausgewählt ist, die aus Brustkrebs, Lungenkrebs und Lymphom besteht.

14. Die Verwendung nach Anspruch 13, wobei genannter Inhibitor in genanntem Individuum die Genexpression von IL-13 oder die Aktivität von IL-13 inhibiert.

15. Die Verwendung nach Anspruch 13, wobei genannter Inhibitor eine IL-13 bindende Region eines IL-13-Rezeptors umfaßt und zusätzlich ein Immunglobulinpolypeptid.

16. Die Verwendung nach Anspruch 13, wobei genannter Antikörper ein polyklonaler Antikörper oder ein monoklonaler Antikörper ist.


**Revendications**

1. Utilisation d'un inhibiteur de IL-13, dans laquelle ledit inhibiteur comprend une région de liaison de IL-13 d'un récepteur de IL-13 ou bien est un anticorps qui se lie à la IL-13, pour la préparation d'une composition pharmaceutique destinée à inhiber la croissance d'une tumeur chez un, sujet, ladite tumeur étant choisie dans le groupe consistant en un cancer du sein, un cancer du poumon et un lymphome.

2. Utilisation suivant la revendication 1, dans laquelle ledit inhibiteur est administré :

   a) en une quantité suffisante pour inhiber la croissance d'une tumeur chez ledit sujet ;
   b) avant détection d'une tumeur primaire chez ledit sujet ;
   c) après détection d'une tumeur primaire chez ledit sujet ;
   d) avant traitement d'une tumeur chez ledit sujet ;
   e) après traitement d'une tumeur chez ledit sujet ;
   f) dans les deux semaines précédant la résection chirurgicale d'une tumeur chez ledit sujet ;
   g) dans les deux semaines suivant la résection chirurgicale d'une tumeur chez ledit sujet;
   h) de manière systémique audit sujet;
   i) de manière topique audit sujet;
   j) par une voie choisie entre les voies d'administration intraveineuse, sous-cutanée et intramusculaire ; ou
   k) dans une composition à libération retardée.

3. Utilisation suivant la revendication 1, dans laquelle ledit sujet présente un risque accru de développer ladite tumeur ou possède un allèle d'un gêne qui augmente le risque de développer ladite tumeur chez ledit sujet.

4. Utilisation suivant la revendication 3, dans laquelle ledit gène est un gène suppresseur de tumeur choisi dans le groupe consistant en un gène de rétinoblastome, un gène p53, un gène BRCA, un gène APC et un gène DCC.

**5.** Utilisation suivant la revendication 2, dans laquelle ledit inhibiteur est administré dans les deux semaines suivant la résection chirurgicale d'une tumeur chez ledit sujet, et dans laquelle ledit inhibiteur est administré conjointement avec le traitement d'une tumeur chez ledit sujet.

**6.** Utilisation suivant la revendication 1, dans laquelle ledit inhibiteur comprend en outre un polypeptide du type immunoglobuline.

**7.** Utilisation suivant la revendication 1, dans laquelle ledit inhibiteur est une protéine de fusion comprenant un polypeptide IL-13R$\alpha$2 murin et une région Fc d'un polypeptide IgG$_1$, ou une protéine de fusion comprenant un polypeptide IL-13R$\alpha$2 humain et une région Fc d'un polypeptide IgG$_1$,

**8.** Utilisation suivant la revendication 1, dans laquelle ledit anticorps est un anticorps polyclonal ou un anticorps monoclonal.

**9.** Utilisation suivant la revendication 1, dans laquelle ledit sujet est un être humain.

**10.** Utilisation suivant la revendication 1, comprenant en outre l'administration audit sujet d'un second agent qui inhibe la croissance tumorale, dans laquelle ledit second agent augmente les taux d'interféron gamma chez ledit sujet.

**11.** Utilisation suivant la revendication 2, dans laquelle ladite composition à libération retardée comprend une microparticule.

**12.** Utilisation suivant la revendication 11, dans laquelle ladite microparticule est une microparticule de poly-acide lactique-co-acide glycolique (PLGA).

**13.** Utilisation d'un inhibiteur non cytotoxique de IL-13 pour la préparation d'une composition pharmaceutique destinée à inhiber la croissance d'une tumeur chez un sujet, dans laquelle ledit inhibiteur comprend une région de liaison de IL-13 d'un récepteur de IL-13 ou bien est un anticorps qui se lie à la IL-13 et ladite tumeur est choisie dans le groupe consistant en un cancer du sein, un cancer du poumon et un lymphome.

**14.** Utilisation suivant la revendication 13, dans laquelle ledit inhibiteur inhibe chez ledit sujet l'expression du gène de IL-13 ou l'activité de la IL-13.

**15.** Utilisation suivant la revendication 13, dans laquelle ledit inhibiteur comprend une région de liaison de IL-13 d'un récepteur de IL-13 et comprend en outre un polypeptide du type immunoglobuline.

**16.** Utilisation suivant la revendication 13, dans laquelle ledit anticorps est un anticorps polyclonal ou un anticorps monoclonal.

Fig. 1a

Fig. 1b

Fig. 2a

Fig. 2b

Fig. 3

Fig. 4a

Fig. 4b

Fig. 5a

Fig. 5b

Fig. 5c

Fig. 6

Fig. 7

Fig. 8a

Fig. 8b

Fig. 8c

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6013258 A
- US 5932218 A

### Non-patent literature cited in the description

- **DONALDSON et al.** *J. Immunol.,* 1998, vol. 161, 2317-24
- **MERRIFIELD.** *J. Amer. Chem. Soc.,* 1963, vol. 85, 2149-2154
- **ROARK et al.** *J. Imm.,* 1998, vol. 160, 3121-27
- **PRUSSIN et al.** *J. Immunol.,* 1997, vol. 159, 5862-70
- **NOBEN-TRAUTH et al.** *Transgenic Res,* 1996, vol. 5, 487-91
- **SCALZO et al.** *Immunogenetics,* 1995, vol. 41, 148-51
- **NOBEN-TRAUTH et al.** *Proc. Natl Acad. Sci. U S A,* 1997, vol. 94, 10838-43
- **KAPLAN et al.** *Immunity,* 1996, vol. 4, 313-9
- **SMILEY et al.** *Science,* 1997, vol. 275, 977-9
- **MATSUI et al.** *J. Immunol.,* vol. 163, 184-193
- **TAKAHASHI et al.** *Proc. Natl. Acad. Sci. USA,* 1988, vol. 85, 3105-09
- **WILDE et al.** *J. Immunol.,* 1983, vol. 131, 2178-2183
- **DONALDSON et al.** *J Immunol,* vol. 161, 2317-24
- **LEO et al.** *Proc Natl Acad. Sci. USA,* 1987, vol. 84, 1374-78
- **CHEN et al.** *J Immunol,* 1997, vol. 159, 2240-9
- **MATSUI et al.** *J. Immunol.,* 1999, vol. 163, 184-193
- **SHIMODA et al.** *Nature,* 1996, vol. 380, 630-3
- **TAKEDA et al.** *Nature,* 1996, vol. 380, 627-30